# EUROPEAN PATENT SPECIFICATION

(11) **EP 1 663 195 B1**
(45) Date of publication and mention of the grant of the patent: **13.02.2008**
(21) Application number: 04728482.3
(22) Date of filing: 20.04.2004
(51) Int. Cl.: A61K 31/198, A61P 17/00, A61P 17/10

(54) **TAURINE BROMAMINE FOR INHIBITING PATHOGENIC BACTERIA AND FUNGI GROWTH AS WELL AS IN A MICROBICIDAL COMPOSITION**
TAURIN BROMAMIN FÜR DIE INHIBIERUNG VON PATHOGENEN BAKTERIEN UND PILZWACHSTUM SOWIE IN EINER MIKROBIZIDEN ZUSAMMENSETZUNG
PROCEDE POUR INHIBER LA CROISSANCE DE BACTERIES ET DE CHAMPIGNONS PATHOGENES ET COMPOSITION MICROBICIDE

(30) Priority: 22.04.2003 PL 35979203; 07.04.2004 PL 36705204
(43) Date of publication of application: 07.06.2006
(73) Proprietor: Marcinkiewicz, Janusz, 30 127 Krakow (PL); Kasprowicz, Andrzej, 30-658 Krakow (PL); Remin, Ewa, 31-112 Krakow (PL); Remin, Andrzej, 31-112 Krakow (PL); Galos-Hudy, Malgorzata, 32-070 Czernichow, Krakow (PL)
(72) Inventor: MARCINKIEWICZ, Janusz, PL-30-127 Krakow (PL); KASPROWICZ, Andrzej, PL-30-658 Krakow (PL)
(74) Representative: Hudy, Ludwik
(86) International application number: PCT/PL2004/000027
(87) International publication number: WO 2004/093853

(56) References cited:
- YAZDANBAKHSH M ET AL: "KILLING OF SCHISTOSOMULA BY TAURINE CHLORAMINE AND TAURINE BROMAMINE" AMERICAN JOURNAL OF TROPICAL MEDICINE & HYGIENE, LAWRENCE, KS, US, vol. 37, no. 1, July 1987 (1987-07), pages 106-110, XP001027193 ISSN: 0002-9637
- MARCINKIEWICZ J ET AL: "TAURINE CHLORAMINE, A PRODUCT OF ACTIVATED NEUTROPHILS, INHIBITS IN VITRO THE GENERATION OF NITRIC OXIDE AND OTHER MACROPHAGE INFLAMMATORY MEDIATORS" JOURNAL OF LEUKOCYTE BIOLOGY, FEDERATION OF AMERICAN SOCIETIES FOR EXPERIMENTAL, US, vol. 58, no. 6, 1 December 1995 (1995-12-01), pages 667-674, XP002036411 ISSN: 0741-5400
- NAGL M ET AL: "TOLERANCE OF N-CHLOROTAURINE, A NEW ANTIMICROBIAL AGENT, IN INFECTIOUS CONJUCTIVITIS - A PHASE II PILT STUDY" OPHTHALMOLOGICA, KARGER, BASEL, CH, vol. 214, no. 2, March 2000 (2000-03), pages 111-114, XP001027188 ISSN: 0030-3755
- OLSZANECKI R ET AL: "Taurine chloramine and taurine bromamine induce heme oxygenase-1 in resting and LPS-stimulated J774.2 macrophages." AMINO ACIDS. AUG 2004, vol. 27, no. 1, August 2004 (2004-08), pages 29-35, XP009037765 ISSN: 0939-4451
- Internet Article [online] Proceedings of the 34th Annual Meeting of the German Society of Immunology, Berlin, Septebmer 24-27, 2003 URL: http:www.wissenschaft-online.de/dgfi/fall0 3/homepage/abstract_detail.php?artikel_id= 1058 XP002299928

## Description

The present invention relates to a method for inhibiting pathogenic bacteria and fungi growth and a microbicidal composition to be applied in the treatment of dermal diseases and associated diseases attributable, among others causative factors to *propionibacterium* types, particularly in the treatment of common acne *(Acne vulgaris)* as well as a composition comprising taurine, and its uses in filed of cosmetics and/or pharmacology, especially dermatology.

The human skin and the external mucosa are constantly in contact with their surrounding environment. Hence they are exposed to diseases caused by bacteria, fungi and viruses. Bacterial skin diseases are mostly caused by mixed staphylococcal/streptococcal infections. Dermatomycoses are pathogen fungi. One of the most common skin diseases is acne in which bacteria *Propionibacterium acnes* is an etiological factor. The acne (A*cne vulgaris*) is experienced during pubertal changes, this condition being associated with the development of hair-follicles, together with their associated sebaceous and excretory duct systems in association with the formation of keratinocytes. Despite the progress in diagnostic techniques, the acne remains a problem, not only for patients, but also for medical diagnostic teams attempting to alleviate the problem. It is assumed that some 85% of the human population experiences these changes, associated with maturation, in a more or less intense form. More aggravated symptoms, at varying levels, occur within the 14-18 year-old age-range, and involve some 10% of the population. Either gender is susceptible to the condition, but it is particularly prevalent in its severest form for pubertal males, due to genetic and hormonal changes.

The etiology of acne is attributable to several causative origins, including; hormonal balances, genetics, immune reactions, and bacterial factors, among others to be mentioned. Juvenile acne occurs as a result of the increase of sebum production by the cutaneous glands under the influence of increased secretion of androgenic hormones during the pubertal phase. In the development of the acne, four factors have primary significance: increased secretion of sebum, excessive keratosis, blockage of the folicular/sebacial duct outlets and invasion and colonization by anaerobic bacteria *(Propionibacterium acnes)* that cause an inflammatory state in and around the sebaceous glands and result in the formation of so-called blackheads, inflamed papulae, surface cysts, atheromatous cysts and pustules. Androgens, as already mentioned, stimulate the secretion of sebum from the sebaceous glands. Sebum is a fatty substance that maintains a proper level of hydration of the skin and in keratin, the latter constituting the principal component of hair. The other main causative factor for the progenesis of acne is the obstruction of the ducts that exude sebum onto the surface of the skin. This leads to enlargement of the ducts of the sebaceous glands and the development firstly of closed comedones, and then to their bursting. The blockage results in the excessive amounts of generated sebum being retained. Blackheads or points with white heads appear. These changes are non-inflammatory in character. If the walls of the overfilled follicle burst, the secretions may be exuded into adjacent tissue-layers, resulting in the development of inflammatory acne. The infection invades the dermal tissue, cysts arise and these may burst at a later stage, leaving transient or even persistent tissue scarring. These changes may become subject to super-infection. The bacterium *P. acnes,* by introducing chemotactic factors, may engender a polynuclear leukocytes inflow to the sebaceous glands. As a result of the leukocytes induced phagocytes of the bacteria, hydrolytic enzymes are liberated, leading to the destruction of the walls of the sebaceous glands, allowing their content to invade the corium and leading to the development of an inflammatory condition. *Papulo pustuar* eruptions may appear. In severe forms of acne, deep, purulent infiltrations and fistulae are generated, leading to local scarring. Within the blocked sebaceous glands the development of anaerobic bacteria takes place. These bacteria produce enzymes that decompose the sebum into free fatty acids, thereby intensifying the inflammatory state. The bacteria *P*. *acnes* and *P. granulosum* are mainly found in areas of the human body rich in sebaceous glands such as the head, the chest, the back, mainly in the interscapular region. The above mentioned factors, associated with a raised concentration of microorganisms, give rise to chronic inflammatory processes, involving the sebaceous hair follicles and the sebaceous glands with which they are associated. Stimulation of the sebaceous glands induces increased production of sebum whose lipid-rich secretions form an excellent medium in which bacteria may breed. A correlation between the severity of the degree of acne and the level of secretion of sebum has been observed. At the same time as the process is taking place, dyskeratinisation occurs - i.e. an abnormal proliferation of ductal keratomycetes and the avulsion of sebaceous gland cells, leading to the blockage of the ducts, thereby creating ideal conditions for the development of anaerobic bacterial growths.

In recognition of the variable etiology of changes associated with acne, the potential increase in the intensity of such changes and the various sites in which the condition may occur, often diverse suggestions have been made as to how to treat this pathological state. The principal aim of treatment for common acne is to arrest the symptoms of seborrhea by constricting the dermal pores, removal of the retained secretions, prevention of the occurrence of comedones, reducing the secretion of sebum, alleviating inflammatory states and decreasing hypersensitivity. Various therapeutic regimes have been applied, such as the administration of hormones (estrogens and pro-estrogens), courses of vitamins (B2, A, C, E, PP) or antibiotics (tetracycline, erythromycin). The traditional treatment uses local applicants containing dehydrative compounds with the addition of salicylic acid, menthol and sulphur, recorcin, exposure to sunlight or UV light (the latter no longer being practiced). The use of salicylic acid is also no longer recommended for the alleviation of the symptoms of acne due to its irritant effect in many cases. As an adjunct to treatment, large doses of vitamins are recommended, particularly those belonging to the vitamin B group. Hormone-based medicines form one of other groups; those which diminish the effect of androgens. Medicines containing progesterones are most frequently prescribed. They are most effective in cases of mild acne. Their action causes a diminution in concentrations of androgens and thereby reduces the rate of sebum secretion and the component creating conditions for inflammation. Spironolactone is another medicine with a similar effect and is popular for the treatment of acne. It has also been demonstrated that corticosteroids given generally, inhibit the secretion of androgens. Corticosteroids are mainly administered by direct injection to isolated deforming acne changes of a cystic or tubercular character to accelerate their rate of healing. Nevertheless, hormone-based medicines are not the most suitable and are only partially effective in cases exhibiting advanced dermal pathological conditions. Additionally, if large doses of Spironolactone (as mentioned earlier) are administered, women may experience menstrual irregularities and men may suffer from gynaecomastia and loss of libido. Dizziness and a general feeling of fatigue may also be experienced.

Retinoids form another group of medicines, being derivatives of Vitamin A. They are applied to slow the process of keratinisation, and are typically applied both locally and generally. Isotretynoine is characterized by its effect in stabilizing abnormal *keratosis pilaris* and inhibiting the process of desquamination of epithelial cells by weakening their interconnections. Among generally applied Retinoids, lsotretynoine has been proved to be the most effective. Its effectiveness relies on its reducing sebum secretion. Simultaneously it normalizes the process of keratinisation in the hair follicles and although it has no direct antibacterial activity its effects, as noted above, result in a decrease in the changes occurring within hair follicles. Treatment with Isotretynoine is particularly effective in severe cases of acne that exhibit inflamed cysts with the proviso that such treatment should be carefully monitored, especially in the case of female patients as it is not recognized as being entirely safe. Such patients should be tested for pregnancy before treatment is commenced, least fetal damage be caused. The treatment is contraindicated in cases of pregnancy. It is also recommended that a course of treatment with Isotretynoine should be accompanied by the use of oral contraceptives by women. The effect of Retinoids is the normalization of the desquamation process in the outlet ducts of the sebaceous glands, thereby facilitating the exudation of gland secretions and thereby resulting in a discernible reduction of comedogenesis. For this reason their main application is in cases of *acne comedonica.* Contraindications are also exhibited by exsiccation, desquamation and reddening of the skin, these being frequent symptoms in patients taking Isotretynoine. The use of such medicines may cause severe undesirable side-effects, particularly teratogenic reactions. Medicines from the Retinoids group, as noted earlier, have no direct bactericidal activity. Such action is desirable in cases requiring treatment for dermal changes. In addition to the adverse effects of therapy using Retinoids, there are observed increases in levels of transaminases, bilirubin and hyper-three-glycerids. For this reason patients in the risk-group i.e. with a family history of hyper-three-glyceridemia or diabetes are advised to be examined both before treatment and two months thereafter, not only for general medical examination but with particular attention being paid to the liver and to lipid levels. Frequently-experienced side effects also include drying of the mucosae of the lips and the nasal cavity and conjunctivitis.

Yet another group of medicines used to treat pathological dermal states are antibiotics. They are normally used for local lavage or are applied locally as gels or ointments as well as being taken orally. The main activity of such medicines lies in their antibacterial effect, thereby diminishing the level of colonization by bacteria of the external pores of the sebaceous glands. By decreasing the number of microorganisms they directly decrease concentrations of free fatty acids, thereby lowering the levels of lipase and protease in the hair follicles. Hence antibiotics are both antibacterial and anti-inflammatory in their action. The main groups of antibiotics used for acne treatment are tetracyclins, macrolids, clindamycin and cotrimoctrisol. The main disadvantage of antibiotics is that they cannot be used for extended periods, their application being restricted to 3 - 4 weeks. Moreover, the excessive, uncontrolled and indiscriminate use of antibiotics and sulphonamides, as was the case in the 1970s and '80s has resulted in the fact that nowadays the use of many chemotherapeutic agents even on the skin has resulted in bacterial immunity to them, thereby rendering such agents ineffective. This concerns, among others, neomycin and tetracycline. There is also the possibility of adverse reactions.

Apart from antibiotics a number of locally-applied agents are used for the treatment of mild acne. These include benzyl peroxide, azelan acid, sulphur preparations and salicylic acid, the latter having been mentioned earlier.

A lesser-known therapy is to inject triamcinolon, a type of steroid directly into the cyst. This may have the temporary side-effect of darkening the skin around the site of the injection.

Adjunctive therapies are also used and these should be preceded by the patient being clearly and comprehensibly informed about them. Individual approach of patients' regimes, possibly administering several medicines simultaneously, in conjunction with hygiene/cosmetic procedures, and if necessary with cryotherapy, lasertherapy or treatment with liquid nitrogen may alleviate the symptoms of acne considerably.

Clinical tests have also been conducted using phototherapy. The promising effects of photodynamic treatment observed after the external use of ALA solution and irradiation of the skin of patients with acne are primarily a decrease in the number of inflammatory changes, the photocytotoxic damage to the sebaceous glands the long-term suppression of sebum production and a considerable decrease in the numbers of bacteria.

In some cases vaccines and autovaccines are used. Irrespective of which is applied an immune reaction occurs identically because all these preparations contain *Propionibacterium acnes* strains with strong immunomodulating activity. The practice indicates, however, that in cases of patients treated with vaccines or autovaccines, there is a need to simultaneously administer preparations with antibacterial and anti-inflammatory activity. On the other hand, it has been stated that treatment with vaccines containing antigens *Propionibacterium acnes* and *Propionibacterium granulosum* turned out to have little effect - only about 30% of patients experienced a limited improvement and the same proportion of patients showed a worsening of their condition. Administering autovaccines, in the light of contemporary knowledge, would seem to be unjustified.

Another treatment of acne is known from the US Patent No. US 4,772,592 that describes a water-in-oil emulsion suitable for topical application to human skin. The water-in-oil emulsion comprises in addition to water a C.sub.1 to C.sub.4 alkyl lactate, a silicone oil ingredient containing a dispersion in a volatile siloxane of a polymer of dimethyl polysiloxane with polyoxyethylene and/or polyoxypropylene side chains. These substances are present in specified quantities. Volatile polar liquids, such as alkyl lactate and alkanol constitute essential components of the emulsion because, in association with silicone oil they act to stabilize it. However, the C.sub.1 to C.sub.4 alkyl lactate irritates the skin and the mucosae.

The publication entitled "Killing of schistosomula by taurine chloramine and taurine bromamine" to Yazdanbakhsh et al., American Journal of Tropical Medicine & Hygine, Lawrence, KS, US, Vol. 37, No. 1, July 1987, pages 106-110, shows that taurine chloramine and taurine bromamine in physiological concentrations are able to kill the schistosomula of *Schistosoma mansoni.* According to this publication are rather stable compounds and can accumulate around a schistosomulum and have a long-lasting effect. In contrast, although taurine bromamine is toxic for schistosomula, it cannot act as a long-lived agent against parasites.

The publication entitled "Taurine chloramines, a product of activated neutrophils, inhibits in vitro the generation of nitric oxide and other macrophage inflammatory mediators" to Marcinkiewicz et al., Journal of Leukocyte Biology, Federation of American societies for experimental, US, Vo. 58, No. 6, 1 December 1995, pages 667-674, teaches that the major function of taurine chloramine in leukocytes is to trap chlorinated oxidants.

The publication entitled "Tolerance of N-chlorotaurine, a new antimicrobial agent, in infectious conjuctivitis - A phase II Pilt study" to Nagl et al.:, Ophthalmologica, Karger, Basel, CH. Vol. 21, No. 2, March 2000, pages 111-114, teaches that N-Chlorotaurine seems to be useful in the treatment of infectious conjunctivitis.

It is an aim of this invention to provide a method for inhibiting pathogenic bacteria and fungi growth that is more effectively than known methods.

It is another object of this invention to provide a bactericidal composition to be applied in the treatment of dermal diseases allowing selectively acting on pathogenic bacteria and fungi.

According to the present invention, taurine bromamine [TauBr] or solution of taurine bromamine [TauBr] is used in the manufacture of a medicament for inhibiting pathogenic bacteria and fungi growth in the treatment of pathological dermal conditions. The therapeutically effective amount of taurine bromamine [TauBr] has concentrations of 10 - 100% content by weight in the solution or the solution with the effective quantity of taurine bromamine [TauBr] has concentrations of 10 - 100% content by weight. A microbicidal, especially bactericidal, composition, according to the invention, contains taurine bromamine [TauBr] being its essential component at concentrations of 10-100% by weight.

The taurine bromamine [TauBr] with cetomacrogol emulsion or methylcellulose gel or powder or glycerol can be used as a pharmaceutical composition or a medicament in the treatment of acne. The taurine bromamine [TauBr] can be a solution in a methylcellulose gel or a cream with pH of 5 - 7.

The composition, besides the taurine bromamine, can contain a lipophyllic phase - liquid paraffin, acetyl alcohol and a hydrophyllic phase - propylene glycol and estolate.

The taurine bromamine [TauBr] may be included as a main ingredient of soap used for disinfecting a human skin.

Preferably, the taurine bromamine [TauBr] solution is a solution of 3.0 to 4.5 mM taurine bromamine [TauBr] in a cetomacrogol cream or a powder suspension of 3.0 to 5.0 mM taurine bromamine [TauBr].

The taurine bromamine [TauBr] solution can contain 65% by volume of 5% methylcellulose solution and 35% by volume of 10 mM Taurine bromamine as well as 50% by volume of 6.7% methylcellulose solution and 50% by volume of 10 mM taurine bromamine.

It is known that taurine chloramine [TauCl] (N-chloro taurine) has bactericidal properties. Its effect has been described in Hyg. Med. 18, 330-326, 1992. During the studies, it has been found, unexpectedly, that taurine bromamine [TauBr] (N-bromo taurine), which shows much stronger bactericidal activity than taurine chloramine [TauCl], produces a bactericidal effect on bacteria of *Propionibacterium* types, which are one of etiological factors of common acne *(Acne vulgaris).* Moreover, it turned out that taurine bromamine does not destroy the whole bacterial flora but acts selectively in certain conditions.

Different methods for producing taurine bromamine are known in present biochemical technology. In order to evaluate its bactericidal properties according to the invention, taurine bromamine was prepared on the day of the clinical test. In this order the solution of 180 mM NaOCl (Aldrich, Germany) was mixed in a phosphate buffer (POCH, Gliwice, Poland) to obtain NaCl solution with a concentration of 60 mM. The 60 mM solution of NaCl was mixed in equal proportions with a 100 mM solution of NaBr (POCH, Gliwice, Poland). NaOBr obtained in this way was diluted in a phosphate buffer (POCH, Gliwice, Poland) with a final concentration of NaOBr not exceeding 10 mM. To obtain taurine bromamine a solution of NaOBr was introduced by drops (whilst stirring very slowly) to the taurine solution *(Sigma,* St. Louis, MO) with a concentration of ca. 100 mM. The concentration of the taurine solution was 8-12 times higher than the concentration of the NaOBr solution, although taurine may be used in higher concentrations - up to 100 times greater. Preferably, while producing taurine bromamine, the concentration of the taurine solution was around 10 times higher than the concentration of the NaOBr solution. This resulted in the taurine bromamine solution having a molar concentration not exceeding 10 mM The molar concentration of the taurine bromamine produced was assessed by measuring the extinction at a wave-length λₘₐₓ= 286 nm, where the molarity of taurine bromamine equals value of extinction in peak 430⁻¹. To produce, for example 10 ml of 4-6 mM TauBr solution, 0.33 ml of 180 mM NaOCl in 0.67 ml of phosphate buffer (pH 7.4) was mixed with 1ml of 100mM NaBr. The NaOBr so obtained was dissolved in 3 ml of phosphate buffer and stirred very gently while 5 ml of 100 mM taurine was introduced in drops. The TauBr solution was tested at pH ranging from 5.0 to 8.0.

For research purposes, two bacterial strains commonly found on the human skin were chosen: *Propionibacterium acnes* (causing acne) and *Staphylococcus epidermidis* (being a part of physiological flora of the skin). Selected microorganisms were lyophilized and kept at room temperature. To prepare strain cultures for immune research the lyophilisate was dissolved in 0.5 ml of 0.9% NaCl solution. The mixture was stirred and seeded on a growth medium. Incubation was conducted in anaerobic conditions at 37°C for 48 - 72 hrs. A suspension of bacteria at a concentration of 5x 10⁹ CFU/ml was then prepared.

In order to assess the microbicidal activity of the TauBr solution, the *Proponibacterium acnes* and the *Staphylococcus epidermidis* bacteria were incubated in closed tubes with TauBr solution. The range of concentrations tested was 1 - 1000 µM. The incubation was conducted at room temperature for 30 minutes. When this process was completed, bacteria were seeded onto a growth medium. After an appropriate period of time the number of developed colonies were counted. Next, estimation of the susceptibility of the *Proponibacterium acnes* and *Staphylococcus epidermidis* bacteria to the agent tested [TauBr] was determined. Analysis of the susceptibility of the strains tested proved the bactericidal activity of TauBr to the *Proponibacterium acnes* for which the inhibitory concentration was below 32 µM (MIC) and to the *Staphylococcus epidermidis* for which the inhibitory concentration was above 125 µM (MIC)

In further test, the microbicidal composition described in the example, in the form of an emulsion, was applied to the patient's skin. The microbicidal composition consisted of: an active substance - TauBr at concentrations of 10 - 50% and a base (emulsion) - made up to 100%. The base (emulsion) contains a 16% lipophyllic phase - liquid paraffin, acetyl alcohol and an 84% hydrophyllic phase - propylene glycol and estolate (or lauryl sulphate). The bactericidal formulation is particularly useful in the treatment of inflammatory skin conditions caused by bacterial or mycotic infections such as acne and skin candidiosis. Further tests proved that the bactericidal activity of TauBr is much stronger than TauCl especially at pH above 7, at which TauCl shows no bactericidal properties in subcytotoxic concentrations for mammalian cells, whereas TauBr destroys 99% of the bacteria tested. Variable susceptibility of the strains exposed to TauBr was found. TauCl shows no such selective bactericidal properties.

In order to conduct further tests to ascertain the antibacterial activity of the formulation containing taurine bromamine and prepared by the method described above, the imprinting method was chosen with use of Rodac plates with an area of 25 cm². For the purposes of study a *Scheadler Agar Base* medium produced by the firm Dico, catalogue number 212189, was used with addition of 5% ram blood. The medium was poured steadily onto the Rodac plates in aseptic conditions until a convex meniscus appeared. Before being applied, the medium was kept in enclosed conditions within a fridge at a temperature of ca. +4 °C and was used in a time not exceeding 7 days from the date of its preparation.

For the purpose of the studies several volunteers were selected - women aged between 23 and 27, who exhibited no discernable clinical pathological changes of the skin surface and who were not taking preparations with antibacterial activity during the studies, neither on the skin surface nor internally.

Tests for the microbicidal activity of taurine bromamine were conducted by performing 5 imprints of the dorsal skin on time at each of the patients examined. The skin area, on which the tests were to be performed, was determined in advance and marked with the numbers 1 - 5. After having marked the place where the test was to be performed, sterile gauze pads of area 4 cm² saturated with different substances were applied to the skin.

At the site marked 1 - a gauze pad saturated with 0.9% inj. solution of NaCl (Polfa Lublin), which was a control substance without antibacterial activity was applied to the skin. At site 2 - a gauze pad saturated with a 4 - 7 mM solution of taurine bromamine in a buffer with pH 5 - pH 6.4 was applied to the skin. At site 3 - a gauze pad saturated with a 4 - 7 mM solution of taurine bromamine in a buffer with pH 6.4 - pH 7.4 was applied to the skin. At site 4 - a gauze pad saturated with a 1% solution of salicylic acid in a buffer with pH 5 and at the site marked 5 - a gauze pad saturated with a 1% solution of salicylic acid in a buffer with pH 7 was applied to the skin. 1% solutions of salicylic acid in buffers with pH 5 and pH 7 are substances which are used routinely in the treatment of acne. At all sites gauze pads were fixed firmly to the skin surface for 30 minutes and then were removed off. 5 minutes after their removal, as the skin was already dry, the Rodac type plates were pressed down so that the site treated with the substance tested was imprinted centrally on the plate. Next the plates were placed in anaerobic conditions using the Gas Pack system produced by the firm Biomerieux and incubated for 5 days at a temperature of 37 °C. After completing the incubation the bacteria growths on the imprinting plates from sites 2 - 5 after treatment with the substance tested on each patient were compared with the control plate applied at site 1. Wherever possible, all the cultivated colonies of relatively aerobic and anaerobic bacteria were counted, to provide a figure for the concentration per cm² of the skin. The studies were repeated four times on the same patients at one-week intervals, which produced similar results. On the basis of the results obtained a considerable diminution in the number of bacteria on the skin area treated with the 4 - 7 mM solution of taurine bromamine in a buffer with pH 5 - pH 7 was recorded for all the patients examined, and the biggest decrease in bacterial concentration, from the uncountable level of ca 10⁴ CFU/ cm² to a level of 10¹ - 10² CFU/cm² was observed on the skin areas treated with a 5 mM solution of taurine bromamine in a buffer with pH 7. Decrease in bacterial concentration on a human skin was also observed when hands were washed with soap containing, for example bromamine taurine and glycerol or stearin.

Further studies of the microbicidal activity of taurine bromamine were conducted using a gel, a cream and a powder. In these tests, applications with a semisolid consistency and plastic features were made, called hereinafter, pharmaceutical or cosmetic preparations or compositions, particularly a taurine bromamine solution in a methylcellulose gel with pH 5 - pH 7, a cetomacrogol cream with pH 5 - pH 7 and a powder suspension in a taurine bromamine solution.

The powder suspension in taurine bromamine solution was prepared on a base of liquid powder changing the proportions of the components by weight. The suspension consisted of, by weight: 14.0 - 60.0 % of 10 mM Taurine bromamine solution, 10.0 - 24.0 % of talc, 14.0 - 40.0 % of glycerol, 5.0 - 30.0 % of distilled water and 0.5 - 2.0 % of menthol. The constituents of the suspension were stirred thoroughly until a homogenous dispersion of the solid phase was obtained.

The taurine bromamine solution in a methylcellulose gel was prepared on a base of methyl-cellulose solution (Methocel 1500 cP) on a sterile phosphate buffer with pH 7.4 by pouring methyl cellulose to approx. one third of the volume of the prepared buffer solution at a temperature of ca. 90°C and mixed so that the powder became completely moistened. The substance prepared in this way was cooled. The remainder of the buffer solution together with the taurine bromamine solution was added, similarly cooled, and was mixed thoroughly then left in a fridge to become clarified.

To prepare taurine bromamine solution in a cream form, (in brief - the preparation on a cream base), a cetomacrogol cream (Cetomacrogol Cream, Sorbolone Cream) was used according to The Extra Pharmacopoeia, Ed. 29. It was modified by introducing a phosphate buffer at pH 7.4 (with its composition KH₂PO₄ i Na₂HPO₄ x 12H₂O) in different amounts to replace distilled water. Next, the chlorocresol in this cream was replaced with a mixture of preservative agents in different amounts. The mixture contained 1.0 - 3.0 g of benzyl alcohol, 120 - 200 mg of methyl-hydroxybenzoate or sodium hydroxybenzoesan and 60 - 100 mg of propylhydroxybenzoate (propyl hydroxybenzoesan). The production of the cream-based preparation began with melting the components of a cetomacrogol cream oil phase (emulsifying wax, liquid paraffin, white petroleum) in a water bath. Then the water phase (a sterile phosphate solution, a taurine bromamine solution, preserving agents) was emulsified in warmth at a temperature of ca. 60°C into the oil phase and stirred until the mixture set.

The preparations based on a cream and gel base and the powder suspension were applied to the skin of the subjects at the places marked with numbers 1 - 5. Each of the preparations was applied to an area of ca. 4 cm² of every patient tested in the following way: at site "1" - a 0.4 - 0.8 mM taurine bromamine solution in a methylcellulose gel with pH 5 - pH 7 was applied to the skin, at site "2" - a suspension of 3.0 - 4.5 mM taurine bromamine in a methylcellulose gel with pH 5 - pH 7, at site "3" - the preparation of 3.0 - 4.5 mM taurine bromamine solution in a cetomacrogol cream with pH 5 - pH 7 and at site "4" - the powder suspension of 3.0 -5.0 mM taurine bromamine was applied

The preparations were left on the skin area for 30 minutes and then the plates were pressed down so that the site exposed to the activity of the various substances was imprinted centrally on the plate. Later, as in case of the taurine bromamine solutions, the plates were placed in anaerobic conditions using the Gas Pack system produced by Firm Biomerieux and incubated for 5 days at a temperature of 37°C. After completing the incubation, the bacterial growth on the imprinting plates, from the sites marked by 1 to 5, which had been treated with the substance tested, were compared. Wherever it was possible, all cultivated colonies of relatively aerobic bacteria and anaerobic bacteria were counted, giving their occurrence per 1 cm² of the skin. The tests were conducted four times on the same people at one-week intervals, which produced similar results. On the basis of the results the most significant diminution in bacterial activity was observed in all patients on the skin areas treated with the suspension of above 3.5 mM taurine bromamine in the cetomacrogol cream at pH 7.

The taurine bromamine solutions in a cream and in a gel and powder suspensions in the taurine bromamine solutions, which may serve as examples of cosmetic compositions and pharmaceutical compositions, are given below. Proportions of the components of the substances mentioned below may be changed in amount of ± 50% by weight in response to the sensitivity of the skin. However, in case of a normal skin it is recommended that the molarity of these substances should not exceed 5 mM taurine bromamine, because above this level all the bacterial flora is destroyed, for example *Staphylococcus epidermidis.*

**Example 1. 100 ml of 3.5 mM modified cream**

| | |
|---|---|
| Cetomacrogol emulsion about | 15.0 ml |
| Liquid paraffin about | 10.0 ml |
| Vaseline (or paraffin jelly) about | 10.0 ml |
| Benzyl alcohol | 1.5 ml |
| Sodium hydroxybenzoesan | 0.15 ml |
| Propyl hydroxybenzoesan | 0.08 ml |
| Propylene glycol | 5.0 ml |
| 10 mM Taurine bromamine | 35.0 ml |
| Water ad | 100.0 ml |

**Example 2. 100 ml of 5.0 mM modified cream**

| | |
|---|---|
| Cetomacrogol emulsion about | 15.0 ml |
| Liquid paraffin about | 10.0 ml |
| Vaseline (or paraffin jelly) about | 10.0 ml |
| Benzyl alcohol | 1.5 ml |
| Sodium hydroxybenzoesan | 0.15 ml |
| Propyl hydroxybenzoesan | 0.08 ml |
| Propylglucose | 5.0 ml |
| 10 mM Taurine bromamine | 50.0 ml |
| Water ad | 100.0 ml |

**Example 3. 100 ml of 3.5 mM modified gel**

| | |
|---|---|
| 5% methylcellulose solution | 65.0 ml |
| 10 mM Taurine bromamine | 35.0 ml |

**Example 4. 100 ml of 5.0 mM modified gel**

| | |
|---|---|
| 6.7% methylcellulose solution | 50.0 ml |
| 10 mM Taurine bromamine | 50.0 ml |

**Example 5. 100 ml of 3.5 mM fluid powder**

| | |
|---|---|
| 10 mM Taurine bromamine | 35.0 ml |
| Talc | 22.0 ml |
| Glycerol | 38.0 ml |
| Menthol | 1.0 ml |
| Distilled water ad | 100.0 ml |

**Example 6. 100 ml of 5.0 mM fluid powder**

| | |
|---|---|
| 10 mM Taurine bromamine | 50.0 ml |
| Talc | 19.0 ml |
| Glycerol | 29.0 ml |
| Menthol | 1.0 ml |
| Distilled water ad | 100.0 ml |

## Claims

1. Use of taurine bromamine [TauBr] or solution of taurine bromamine [TauBr] in the manufacture of a medicament for inhibiting pathogenic bacteria and fungi growth in the treatment of pathological dermal conditions.

2. Use of the taurine bromamine or the solution of the taurine bromamine according to claim 1 **characterized in that** effective quantity of taurine bromamine [TaurBr] in the solution is 10 - 100% by weight

3. Use of the taurine bromamine or the solution of the taurine bromamine according to claim 1 **characterized in that** the solution is a solution of taurine bromamine [TaurBr] at concentration 10 - 100% of taurine bromamine [TaurBr] by weight.

4. Use of the taurine bromamine [TauBr] or the solution of taurine bromamine [TauBr] according to claim 1 **characterized in that** the solution is 4 - 7 mM solution of taurine bromamine [TauBr].

5. Use of the taurine bromamine [TauBr] or the solution of the taurine bromamine [TauBr] according to claim 1 **characterized in that** the taurine bromamine [TauBr] is a main ingredient of soap used for disinfecting a human skin.

6. Use of the taurine bromamine [TauBr] or the solution of taurine bromamine [TauBr] according to claim 1 **characterized in that** the medicament is used in treatment of acne.

7. Use of the taurine bromamine [TauBr] or the solution of the taurine bromamine [TauBr] according to claim 1 **characterized in that** the taurine bromamine [TauBr] solution is a solution in a methylcellulose gel with pH 5 - pH 7.

8. Use of the taurine bromamine [TauBr] or the solution of the taurine bromamine [TauBr] according to claim 1 **characterized in that** the taurine bromamine [TauBr] solution is a solution in a cetomacrogol cream with pH 5 - pH 7.

9. Use of the taurine bromamine [TauBr] or the solution of the taurine bromamine [TauBr] according to claim 1 **characterized in that** the taurine bromamine [TauBr] solution is a powder suspension in the taurine bromamine solution.

10. Use of the taurine bromamine [TauBr] or the solution of the taurine bromamine [TauBr] according to claim 1 **characterized in that** the taurine bromamine [TauBr] with glycerol constitutes a basis of pharmaceutical composition.

11. Use of the taurine bromamine [TauBr] or the solution of the taurine bromamine [TauBr] according to claim 1 **characterized in that** the taurine bromamine [TauBr] with cetomacrogol emulsion constitutes a basis of pharmaceutical composition.

12. Use of the taurine bromamine [TauBr] or the solution of the taurine bromamine [TauBr] according to claim 1 **characterized in that** the taurine bromamine [TauBr] with methylcellulose solution constitutes a basis of pharmaceutical composition.

13. Use of the taurine bromamine [TauBr] or the solution of the taurine bromamine [TauBr] according to claim 1 **characterized in that** the solution of the taurine bromamine [TauBr] contains a lipophyllic phase-liquid paraffin, acetyl alcohol and an hydrophilic phase-propylene glycol and estolate.

14. Use of the taurine bromamine [TauBr] or the solution of the taurine bromamine [TauBr] according to claim 1 **characterized in that** the taurine bromamine [TauBr] solution is prepared on a base of methylcellulose solution.

15. Use of the taurine bromamine [TauBr] or the solution of the taurine bromamine [TauBr] according to claim 1 **characterized in that** the taurine bromamine [TauBr] solution is a solution of 3.0 to 4.5 mM taurine bromamine [TauBr] in a cetomacrogol cream.

16. Use of the taurine bromamine [TauBr] or the solution of the taurine bromamine [TauBr] according to claim 1 **characterized in that** the taurine bromamine [TauBr] solution is a powder suspension of 3.0 to 5.0 mM taurine bromamine [TauBr].

17. Use of the taurine bromamine [TauBr] or the solution of the taurine bromamine [TauBr] according to claim 1 **characterized in that** the taurine bromamine [TauBr] solution contains 65% by volume of 5% methylcellulose solution and 35% by volume of 10 mM Taurine bromamine.

18. Use of the taurine bromamine [TauBr] or the solution of the taurine bromamine [TauBr] according to claim 1 **characterized in that** the taurine bromamine [TauBr] solution contains 50% by volume of 6.7% methylcellulose solution and 50% by volume of 10 mM Taurine bromamine.

## Patentansprüche

1. Anwendung von Taurin Bromamin [TauBr] beziehungsweise einer Lösung von Taurin Bromamin [TauBr] zur Herstellung eines Arzneimittels für die Inhibierung von pathogenen Bakterien und Pilzwachstum bei der Behandlung von Hautkrankheiten.

2. Anwendung von Taurin Bromamin beziehungsweise einer Lösung von Taurin Bromamin [TauBr] nach Anspruch 1 **dadurch gekennzeichnet, daß** die effektive Anzahl von Taurin Bromamin [TauBr] in der Lösung gewichtsmässig 10-100% beträgt.

3. Anwendungen von Taurin Bromamin beziehungsweise einer Lösung von Taurin Bromamin [TauBr] nach Anspruch 1 **dadurch gekennzeichnet, daß** die Lösung eine Lösung von Taurin Bromamin [TauBr] darstellt mit einer Konzentration von Taurin Bromamin [TauBr] gewichtsmässig 10-100 % beträgt.

4. Anwendungen von Taurin Bromamin [TauBr] beziehungsweise einer Lösung von Taurin Bromamin [TauBr] nach Anspruch 1 **dadurch gekennzeichnet, daß** die Lösung eine Lösung von Taurin Bromamin [TauBr] gewichtsmässig 4 - 7 mM beträgt.

5. Anwendung von Taurin Bromamin [TauBr] beziehungsweise einer Lösung von Taurin Bromamin [TauBr] nach Anspruch 1 **dadurch gekennzeichnet, daß** Taurin Bromamin [TauBr] Hauptbestandteil von Seife darstellt, die zur Desinfizierung der menschlichen Haut verwendet wird.

6. Anwendung von Taurin Bromamin [TauBr] beziehungsweise einer Lösung von Taurin Bromamin [TauBr] nach Anspruch 1 **dadurch gekennzeichnet, daß** das Arzneimittel zur Behandlung von Akne verwendet wird.

7. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] eine Lösung darstellt in einem Methyl-Zellulose-Gel mit einem PH-Wert von pH 5 bis pH 7.

8. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] eine Lösung darstellt in einer Cetomacrogolcreme mit einem PH-Wert von pH 5 bis pH 7.

9. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] eine Pudersuspension darstellt in einer Lösung von Taurin Bromamin [TauBr] ist.

10. Anwendung von Taurin Bromamin [TauBr] beziehungsweise einer Lösung von Taurin Bromamin [TauBr] nach Anspruch 1 **dadurch gekennzeichnet, daß** die Taurin Bromamin [TauBr] mit Glycerol Grundlage für eine pharmazeutische Komposition darstellt.

11. Anwendung von Taurin Bromamin [TauBr] beziehungsweise einer Lösung von Taurin Bromamin [TauBr] nach Anspruch 1 **dadurch gekennzeichnet, daß** die Taurin Bromamin [TauBr] mit Cetomakrogolemulsion Grundlage für eine pharmazeutische Komposition darstellt.

12. Anwendung von Taurin Bromamin [TauBr] beziehungsweise einer Lösung von Taurin Bromamin [TauBr] nach Anspruch 1 **dadurch gekennzeichnet, daß** Taurin Bromamin [TauBr] mit Methylzelluloselösung Grundlage für eine pharmazeutische Komposition darstellt.

13. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] Lipophylic Paraffin in flüssigen Aggregatzustand, Acethyl Alkohol, Glykol im hydrophilen Aggregatzustand und Estolate enthält.

14. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] auf der Grundlage von Methylzelluloselösung vorbereitet ist.

15. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] eine Lösung von 3.0 - 6,0 mM von Taurin Bromamin [TauBr] darstellt in einer Cetomacrogolcreme ist.

16. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] eine Pudersuspension von 3.0 bis 5.0 mM Taurin Bromamin [TauBr] darstellt.

17. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] 65 Volumenprozent von 5% Methylzelluloselösung und 35 Volumenprozent von 10 mM Taurin Bromamin [TauBr] enthält.

18. Anwendung von Taurin Bromamin [TauBr] beziehungsweise einer Lösung von Taurin Bromamin [TauBr] nach Anspruch 1 **dadurch gekennzeichnet, daß** die Lösung von Taurin Bromamin [TauBr] 50 Volumenprozent von 6.7% Methylzelluloselösung und 50 Volumenprozent von 10 mM Taurin Bromamin [TauBr] enthält.

## Revendications

1. Utilisation de la bromamine taurine [TauBr] ou de la solution de taurine bromamine [TauBr] pour la manufacture d'un médicament destiné pour inhiber la croissance de bactéries et de champignons pathogènes dans le traitement de la pathologie de la peau.

2. Utilisation de la bromamine taurine [TauBr] ou de la solution de taurine bromamine [TauBr] selon la revendication 1 **caractérisée en ce que** la quantité effective de taurine bromamine [TaurBr] dans la solution est de 10 - 100% en poids.

3. Utilisation de la bromamine taurine [TauBr] ou de la solution de taurine bromamine [TauBr] selon la revendication 1 **caractérisée en ce que** la solution est une solution de taurine bromamine [TauBr] contenant 10 - 100% de taurine bromamine [TauBr] en poids.

4. Utilisation de la bromamine taurine [TauBr] ou de la solution de taurine bromamine [TauBr] selon la revendication 1 **caractérisée en ce que** la solution est une solution de taurine bromamine [TauBr] de 4 - 7 mM.

5. Utilisation de la bromamine taurine [TauBr] ou de la solution de taurine bromamine [TauBr] selon la revendication 1 **caractérisée en ce que** la taurine bromamine [TauBr] est le composant essentiel d'un savon destiné à la désinfection de la peau humaine.

6. Utilisation de la bromamine taurine [TauBr] ou de la solution de taurine bromamine [TauBr] selon la revendication 1 **caractérisée en ce que** un médicament est destiné au traitement acné.

7. Utilisation de la bromamine taurine [TauBr] ou de la solution de taurine bromamine [TauBr] selon la revendication 1 **caractérisée en ce que** la solution de taurine bromamine [TauBr] est une solution dans un gel méthylcellulosique au pH 5 - pH 7.

8. Utilisation de la bromamine taurine [TauBr] ou de la solution de taurine bromamine [TauBr] selon la revendication 1 **caractérisée en ce que** la solution de taurine bromamine [TauBr] est un solution dans une crème cetomacrogolique au pH 5 - pH 7.

9. Utilisation de la bromamine taurine [TauBr] ou de la solution de taurine bromamine [TauBr] selon la revendication 1 **caractérisée en ce que** la solution de taurine bromamine [TauBr] est une suspension poudreuse dans la solution de taurine bromamine [TauBr].

10. Utilisation de la bromamine taurine [TauBr] ou de la solution de taurine bromamine [TauBr] selon la revendication 1 **caractérisée en ce que** la taurine bromamine [TauBr] avec le glycérol forme la base d'une composition pharmaceutique.

11. Utilisation de la bromamine taurine [TauBr] ou de la solution de taurine bromamine [TauBr] selon la revendication 1 **caractérisée en ce que** la taurine bromamine [TauBr] avec une émulsion cetomacrogolique forme la base d'une composition pharmaceutique.

12. Utilisation de la bromamine taurine [TauBr] ou de la solution de taurine bromamine [TauBr] selon la revendication 1 **caractérisée en ce que** la taurine bromamine [TauBr] avec une solution méthylcellulosique forme la base d'une composition pharmaceutique.

13. Utilisation de la bromamine taurine [TauBr] ou de la solution de taurine bromamine [TauBr] selon la revendication 1 **caractérisée en ce que** la solution de taurine bromamine [TauBr] comprend la paraffine en phase liquide lipophyllique, l'alcool acétylique, le glycol en phase propylène hydrophilique et l'estolate.

14. Utilisation de la bromamine taurine [TauBr] ou de la solution de taurine bromamine [TauBr] selon la revendication 1 **caractérisée en ce que** la solution de taurine bromamine [TauBr] est une solution préparée sur la base d'une solution méthylcellulosique.

15. Utilisation de la bromamine taurine [TauBr] ou de la solution de taurine bromamine [TauBr] selon la revendication 1 **caractérisée en ce que** la solution de taurine bromamine [TauBr] est une solution de taurine bromamine de 3.0 - 4.5 mM dans une crème cetomacrogolique.

16. Utilisation de la bromamine taurine [TauBr] ou de la solution de taurine bromamine [TauBr] selon la revendication 1 **caractérisée en ce que** la solution de la taurine bromamine [TauBr] est une suspension poudreuse de taurine bromamine [TauBr] de 3.0 - 5.0 mM.

17. Utilisation de la bromamine taurine [TauBr] ou de la solution de taurine bromamine [TauBr] selon la revendication 1 **caractérisée en ce que** la solution de taurine bromamine [TauBr] comprend une solution de 65% en volume de méthylcellulose à 5% et de 35% en volume de taurine bromamine de 10 mM.

18. Utilisation de la bromamine taurine [TauBr] ou de la solution de taurine bromamine [TauBr] selon la revendication 1 **caractérisée en ce que** la solution de la taurine bromamine [TauBr] comprend une solution de 50% en volume de méthylcellulose à 6.7% et de 50% en volume de taurine bromamine de 10 mM.
